# EUROPEAN PATENT APPLICATION

(11) **EP 4 650 440 A1**
(43) Date of publication of application: **19.11.2025**
(21) Application number: 24755186.4
(22) Date of filing: 14.02.2024
(51) Int. Cl.: C12N 5/10, C12N 15/85, C12P 21/08, C07K 16/46, C12N 15/12

(54) **METHOD FOR PRODUCING CELL, METHOD FOR PRODUCING MULTISPECIFIC ANTIBODY, VECTOR SET, MAMMALIAN CELL, CHO CELL, AND METHOD FOR PRODUCING CELL POOL**

(30) Priority: 17.02.2023 JP 2023023760
(71) Applicant: FUJIFILM Corporation, Tokyo 106-8620 (JP)
(72) Inventor: KURODA, Akari, Ashigarakami-gun, Kanagawa 258-8577 (JP); SEKO, Kenta, Ashigarakami-gun, Kanagawa 258-8577 (JP); MATSUURA, Tatsuya, Ashigarakami-gun, Kanagawa 258-8577 (JP)
(74) Representative: Hoffmann Eitle
(86) International application number: PCT/JP2024/005086
(87) International publication number: WO 2024/172083

(57) **Abstract**

A vector set is introduced into a host cell, and a cell that produces a multispecific antibody is selected from the host cell into which the vector set has been introduced. The vector set is a set of a first expression vector and a second expression vector, in which the first expression vector contains an expression cassette of a first H chain, an expression cassette of a second H chain, an expression cassette of a first L chain, and an expression cassette of a second L chain, and the second expression vector contains one type, two types, or three types of expression cassettes selected from the group consisting of the expression cassette of the first H chain, the expression cassette of the second H chain, the expression cassette of the first L chain, and the expression cassette of the second L chain.

## Description

### BACKGROUND OF THE INVENTION

### 1. Field of the Invention

The present disclosure relates to a method for producing a cell, a method for producing a multispecific antibody, a vector set, a mammalian cell, a CHO cell, and a method for producing a cell pool.

### 2. Description of the Related Art

JP2020-509754A discloses a method for producing a multispecific antibody containing at least three different polypeptides, the method including transfecting a mammalian cell with a first expression vector and one type, two types, or three types of further expression vectors.

JP2012-179051A discloses an expression vector and an expression method for expressing a humanized diabody-type bispecific antibody that is a bispecific antibody targeting HER1 and CD3 and is composed of two types of single-chain polypeptides.

### SUMMARY OF THE INVENTION

In the related art, for the purpose of producing a cell that produces a multispecific antibody, an expression vector for a polypeptide constituting the multispecific antibody has been introduced into a host cell. In a case where the multispecific antibody has four types of polypeptides, for example, an expression vector containing all four types of expression cassettes is introduced into a host cell. However, in reality, the expression level or the purity of the multispecific antibody is low, and the amount of the multispecific antibody produced may not reach the expected value. The reasons for this are considered to be that the transcription rate or the translation rate varies for each polypeptide; some polypeptides are easily decomposed; and all polypeptides aggregate in a case where the number of a specific type of polypeptide is excessive.

Furthermore, the following reasons are also considered to causes of the low expression level or the low purity of the multispecific antibody.

Since the multispecific antibody has a plurality of types of H chains and/or L chains, a mispairing between the H chain and the L chain (a pair that is not intended by the designer of the multispecific antibody and does not obtain the expected antibody activity) may be formed. To efficiently form a target multispecific antibody, a technique of modifying an amino acid sequence of an H chain and/or an L chain (for example, a Knobs-into-holes technique, or an amino acid substitution for the purpose of controlling a charge at an interface between a constant region of an H chain and a constant region of an L chain) may be applied, but it is known that the occurrence rate of mispairing varies depending on the technique used. Since the occurrence of mispairing cannot be completely suppressed even in a case where the above-described technique is applied, the optimal expression level ratio of each polypeptide for obtaining a target multispecific antibody is not necessarily the same as the number ratio of each polypeptide contained in the multispecific antibody.

As a means for increasing the expression level or the purity of the multispecific antibody, it is conceivable to insert each expression cassette of each polypeptide into a separate expression vector and introduce a plurality of types of expression vectors into a host cell. In a case where the multispecific antibody has four types of polypeptides, four types of expression vectors are introduced into a host cell. Since the number of each of the four types of expression vectors to be introduced into the host cell varies for each host cell, diversity occurs in the number of each expression cassette carried by the host cell. A cell having a high expression level or high purity of the multispecific antibody is selected from various host cells. However, it is necessary to construct expression vectors of the same number of types as the number of types of polypeptides constituting the multispecific antibody, and it is necessary to prepare selection markers of the same number of types as the number of types of expression vectors.

As another means for increasing the expression level or the purity of the multispecific antibody, it is conceivable to know in advance the optimal ratio of the expression levels of all the polypeptides constituting the multispecific antibody, and to insert expression cassettes of all types of polypeptides into one expression vector in the number ratio according to this ratio. However, in a case where the multispecific antibody consists of four types of polypeptides, it is not always easy to know the optimal ratio of the expression levels of the four types of polypeptides. In addition, in a case where the optimal ratio of the expression levels of the four types of polypeptides is a relatively large integer (for example, 1:1:2:3), the total number of expression cassettes inserted into one expression vector increases. As a result, the size of the expression vector increases, and the introduction rate into the host cell decreases.

The present disclosure provides a method different from the above-described method as a means of increasing the expression level or the purity of the multispecific antibody.

An object of the present disclosure is to provide a method for producing a cell having excellent productivity of a multispecific antibody.

An object of the present disclosure is to provide a method for producing a multispecific antibody having excellent productivity.

An object of the present disclosure is to provide a vector set used for producing a cell that produces a multispecific antibody.

An object of the present disclosure is to provide a mammalian cell and a CHO cell that produce a multispecific antibody.

An object of the present disclosure is to provide a method for producing a cell pool used for selecting a cell that produces a multispecific antibody.

In the method for producing a cell according to the present disclosure, a first expression vector and a second expression vector, that is, two expression vectors are introduced into a host cell to express a multispecific antibody having four types of polypeptides.

All expression cassettes of four types of polypeptides constituting the multispecific antibody are inserted into the first expression vector. The expression of the four types of polypeptides is achieved by introducing the first expression vector into the host cell.

An expression cassette of a polypeptide of interest (generally, a polypeptide whose expression is desired to be enhanced) among the four types of polypeptides constituting the multispecific antibody is inserted into the second expression vector. The polypeptide of interest is one type, two types, or three types. By introducing the second expression vector into the host cell in addition to the first expression vector, the number of copies of the expression cassette of the polypeptide of interest that is introduced into the host cell increases relatively, and the expression of the polypeptide of interest is enhanced.

Since the number of the first expression vectors to be introduced into the host cell and the number of the second expression vectors to be introduced into the host cell vary for each host cell, diversity occurs in the number of each expression cassette carried by the host cell. A cell that satisfies the evaluation criteria (for example, the expression level or the purity of the multispecific antibody) is selected from among various host cells.

The specific methods for achieving the object include the following aspects.
<1> A method for producing a cell which is a method for producing a cell that produces a multispecific antibody having four types of polypeptides, that is, a first H chain, a second H chain, a first L chain, and a second L chain, the method comprising:
   introducing the following vector set into a host cell; and
   selecting the cell that produces the multispecific antibody from the host cell into which the vector set has been introduced,
   the vector set: a set of a first expression vector and a second expression vector, in which the first expression vector contains an expression cassette of the first H chain, an expression cassette of the second H chain, an expression cassette of the first L chain, and an expression cassette of the second L chain, and the second expression vector contains one type, two types, or three types of expression cassettes selected from the group consisting of the expression cassette of the first H chain, the expression cassette of the second H chain, the expression cassette of the first L chain, and the expression cassette of the second L chain.
<2> The method for producing a cell according to <1>, in which the second expression vector contains the expression cassette of the first L chain and the expression cassette of the second L chain.
<3> The method for producing a cell according to <1> or <2>, in which the multispecific antibody is a pentamer in which the first L chain and the second L chain are bound to the first H chain and the second L chain is bound to the second H chain.
<4> The method for producing a cell according to any one of <1> to <3>, in which the first expression vector contains one copy each of the expression cassette of the first H chain, the expression cassette of the second H chain, the expression cassette of the first L chain, and the expression cassette of the second L chain, and the second expression vector contains one copy each of one type, two types, or three types of expression cassettes selected from the group consisting of the expression cassette of the first H chain, the expression cassette of the second H chain, the expression cassette of the first L chain, and the expression cassette of the second L chain.
<5> The method for producing a cell according to any one of <1> to <4>, in which all of expression cassettes of polypeptides constituting the multispecific antibody contain the same type of promoter.
<6> The method for producing a cell according to any one of <1> to <5>, in which the first expression vector further contains an expression cassette of a first selection marker, and the second expression vector further contains an expression cassette of a second selection marker.
<7> The method for producing a cell according to any one of <1> to <6>, in which the host cell is a mammalian cell.
<8> The method for producing a cell according to any one of <1> to <6>, in which the host cell is a CHO cell.
<9> A method for producing a multispecific antibody, comprising:
   culturing a cell produced by the method for producing a cell according to any one of <1> to <8>.
<10> A vector set that expresses a multispecific antibody having four types of polypeptides, that is, a first H chain, a second H chain, a first L chain, and a second L chain, in which the vector set is a set of a first expression vector and a second expression vector, the first expression vector contains an expression cassette of the first H chain, an expression cassette of the second H chain, an expression cassette of the first L chain, and an expression cassette of the second L chain, and the second expression vector contains one type, two types, or three types of expression cassettes selected from the group consisting of the expression cassette of the first H chain, the expression cassette of the second H chain, the expression cassette of the first L chain, and the expression cassette of the second L chain.
<11> The vector set according to <10>, in which the second expression vector contains the expression cassette of the first L chain and the expression cassette of the second L chain.
<12> The vector set according to <10> or <11>, in which the multispecific antibody is a pentamer in which the first L chain and the second L chain are bound to the first H chain and the second L chain is bound to the second H chain.
<13> The vector set according to any one of <10> to <12>, in which the first expression vector contains one copy each of the expression cassette of the first H chain, the expression cassette of the second H chain, the expression cassette of the first L chain, and the expression cassette of the second L chain, and the second expression vector contains one copy each of one type, two types, or three types of expression cassettes selected from the group consisting of the expression cassette of the first H chain, the expression cassette of the second H chain, the expression cassette of the first L chain, and the expression cassette of the second L chain.
<14> The vector set according to any one of <10> to <13>, in which all of expression cassettes of polypeptides constituting the multispecific antibody contain the same type of promoter.
<15> The vector set according to any one of <10> to <14>, in which the first expression vector further contains an expression cassette of a first selection marker, and the second expression vector further contains an expression cassette of a second selection marker.
<16> A mammalian cell transfected with the vector set according to any one of <10> to <15>.
<17> A CHO cell transfected with the vector set according to any one of <10> to <15>.
<18> A method for producing a cell pool, comprising:
   introducing the vector set according to any one of <10> to <15> into a host cell.
<19> The method for producing a cell pool according to <18>,
   wherein the host cell is a mammalian cell.
<20> The method for producing a cell pool according to <18>,
   wherein the host cell is a CHO cell.

According to the present disclosure, there is provided a method for producing a cell having excellent productivity of a multispecific antibody.

According to the present disclosure, there is provided a method for producing a multispecific antibody having excellent productivity.

According to the present disclosure, there is provided a vector set used for producing a cell that produces a multispecific antibody.

According to the present disclosure, there is provided a mammalian cell and a CHO cell that produce a multispecific antibody.

According to the present disclosure, there is provided a method for producing a cell pool used for selecting a cell that produces a multispecific antibody.

### BRIEF DESCRIPTION OF THE DRAWINGS

Fig. 1 is a schematic diagram of a multispecific antibody that is a tetramer and has four polypeptides.
Fig. 2 is a schematic diagram of a multispecific antibody that is a pentamer and has four polypeptides.
Fig. 3 is a vector map of an expression vector 1 constructed in Examples.
Fig. 4 is a vector map of an expression vector 2 constructed in Examples.

### DESCRIPTION OF THE PREFERRED EMBODIMENTS

Hereinafter, embodiments according to the present disclosure will be described. These descriptions and Examples are only illustrative of the embodiments and do not limit the ranges of the embodiments. The action mechanism mentioned in the present disclosure includes estimation, and the accuracy thereof does not limit the ranges of the embodiments.

In the present disclosure, a numerical range expressed using "to" indicates a range including numerical values before and after "to" as a minimum value and a maximum value.

In the numerical ranges described stepwise in the present disclosure, the upper limit value or the lower limit value described in one numerical range may be replaced with the upper limit value or the lower limit value of the numerical range described stepwise in other stages. In addition, in a numerical range described in the present disclosure, an upper limit value or a lower limit value described in the numerical range may be replaced with a value described in an example.

In the present disclosure, each component may contain a plurality of types of substances corresponding thereto. In the present disclosure, upon referring to an amount of each component in a composition, the amount means a total amount of a plurality of types of substances present in the composition unless otherwise specified, in a case where a plurality of types of substances corresponding to each component are present in the composition.

In the present disclosure, the nucleic acid is a term including any nucleic acid (for example, DNA, RNA, an analog thereof, a natural product, or an artificial product) and a nucleic acid in which a low-molecular-weight compound, a group (for example, a methyl group), a molecule other than a nucleic acid, a structure, or the like is linked to any nucleic acid. The nucleic acid may be single-stranded or double-stranded.

In the present disclosure, a vector is a substance having an action of carrying an exogenous nucleic acid into a cell, and the vector itself is a nucleic acid. The expression vector means a vector that expresses a polypeptide based on the sequence of the nucleic acid. The size and the base sequence of the vector and the expression vector are not limited. The vector and the expression vector are preferably double-stranded DNAs.

In the present disclosure, in the notation of an amino acid, the three-letter notation and the one-letter notation established by IUPAC-IUBMB joint commission on Biochemical Nomenclature (IUPAC-IUBMB JCBN) are used. Unless otherwise specified, the amino acid referred to in the present disclosure is an L-amino acid.

In the present disclosure, the polypeptide is a term including a protein. The number of amino acid residues of the polypeptide is not limited. The polypeptide includes a polypeptide in which an amino acid is post-translationally modified. Examples of the post-translational modification of an amino acid include phosphorylation, methylation, acetylation, glycosylation, lipidation, and the like.

In the present disclosure, one polypeptide means a molecule expressed by one coding sequence that is independently expressed. A relatively large polypeptide (for example, a heavy chain of an antibody) in which a plurality of domains are linked by one or a plurality of peptide linkers is also one polypeptide as long as it is expressed by one coding sequence that is independently expressed.

In the present disclosure, the antibody is not limited to an immunoglobulin and may be any molecule that binds to an antigen. In the present disclosure, the antibody is a term includes an antibody fragment and an antigen-binding molecule. In the present disclosure, the heavy chain of the antibody is also referred to as an H chain, and the light chain of the antibody is also referred to as an L chain.

The present disclosure relates to a multispecific antibody having four types of polypeptides, that is, a first H chain, a second H chain, a first L chain, and a second L chain. Unless otherwise specified, the multispecific antibody mentioned in the present disclosure is the above-described multispecific antibody.

Hereinafter, in a case of describing common matters between the first H chain and the second H chain, both are collectively referred to as an H chain, and in a case of describing common matters between the first L chain and the second L chain, both are collectively referred to as an L chain.

The structure of the multispecific antibody and the number of epitope binding sites are not limited. The multispecific antibody may be a multimer consisting of four polypeptides (that is, a tetramer) or a multimer consisting of five or more polypeptides (that is, a pentamer or a higher multimer).

The multispecific antibody that is a tetramer has one copy each of the first H chain, the second H chain, the first L chain, and the second L chain.

The multispecific antibody that is a pentamer or a higher multimer has at least one first H chain, at least one second H chain, at least one first L chain, and at least one second L chain, in which the number of any chain (may be one type or two or more types) is two or more.

The first H chain and the second H chain are two types of heavy chains having amino acid sequences different from each other. The first H chain and the second H chain are each only required to include at least a region for recognizing an antigen and a region for forming a multispecific antibody. The arrangement of domains in the H chain is not limited to VH-CH1-CH2-CH3, and some domains may overlap or be deleted. The first H chain and the second H chain are connected by a disulfide bond.

The first L chain and the second L chain are two types of light chains having amino acid sequences different from each other. The first L chain and the second L chain are each only required to include at least a region for recognizing an antigen and a region for forming a multispecific antibody. The L chain is connected to the H chain by a disulfide bond.

The multispecific antibody having four types of polypeptides is expressed by four types of coding sequences that are independently expressed, that is, a coding sequence of the first H chain, a coding sequence of the second H chain, a coding sequence of the first L chain, and a coding sequence of the second L chain.

Examples of the multispecific antibody include a human antibody having a human variable region and a human constant region; a mouse antibody having a mouse variable region and a mouse constant region; a chimeric antibody in which regions derived from a plurality of types of animals are combined; a humanized antibody having a mouse variable region and a human constant region; a humanized antibody having a heavy chain variable region of a Camelidae animal and a human Fc region; and the like.

Examples of the multispecific antibody include a bispecific antibody and a trispecific antibody. The bispecific antibody means an antibody that can simultaneously bind to two types of epitopes. The trispecific antibody means an antibody that can simultaneously bind to three types of epitopes. The structure of the bispecific antibody and the number of epitope binding sites are not limited. The structure of the trispecific antibody and the number of epitope binding sites are not limited.

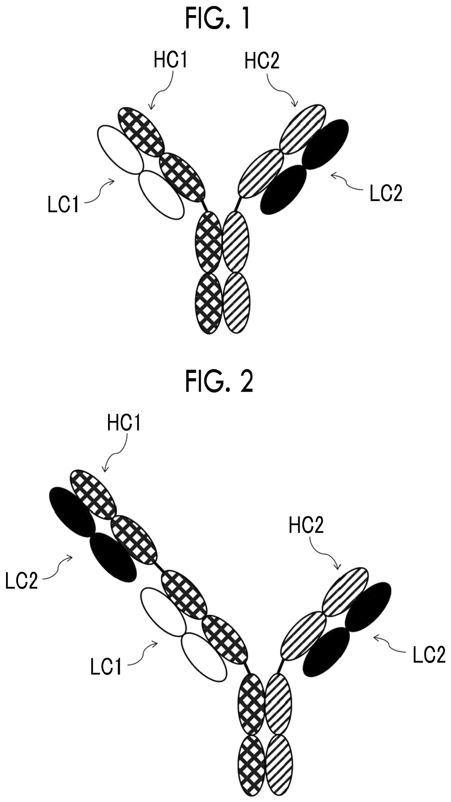

An antibody having a form shown in Fig. 1 is given as an example of the multispecific antibody. The antibody of the present form is a tetramer having one first H chain (HC1), one second H chain (HC2), one first L chain (LC1), and one second L chain (LC2). The antibody of the present form has a form in which LC1 is bound to HC1 and LC2 is bound to HC2. In the antibody of the present form, HC1 and LC1 constitute a first epitope binding site, and HC2 and LC2 constitute a second epitope binding site. The antibody of the present form is a bispecific antibody that binds to a first antigen by a first epitope binding site and binds to a second antigen by a second epitope binding site.

Examples of the bispecific antibody having the form shown in Fig. 1 include Faricimab, Vanucizumab, Mosunetuzumab, and Amivantamab.

An antibody having a form shown in Fig. 2 is given as an example of the multispecific antibody. The antibody of the present form is a pentamer having one first H chain (HC1), one second H chain (HC2), one first L chain (LC1), and two second L chains (LC2). The arrangement of domains in HC1 is VH-CH1-VH-CH1-CH2-CH3. The antibody of the present form has a form in which LC1 and LC2 are bound to HC1 and LC2 is bound to HC2. In the antibody of the present form, HC1 and LC1 constitute a first epitope binding site, HC1 and LC2 constitute a second epitope binding site, and HC2 and LC2 constitute a third epitope binding site. The antibody of the present form may be any of a bispecific antibody that binds to the first antigen by the first epitope binding site and binds to the second antigen by the second epitope binding site and/or the third epitope binding site; or a trispecific antibody that binds to the first antigen by the first epitope binding site, binds to the second antigen by the second epitope binding site, and binds to the third antigen by the third epitope binding site.

Examples of the bispecific antibody having the form shown in Fig. 2 include Cibisatamab, Glofitamab, and Alnuctamab.

### <Method for producing cell>

One of the embodiments of the present disclosure is a method for producing a cell that produces a multispecific antibody. The method for producing a cell according to the present disclosure includes introducing a vector set of a first expression vector and a second expression vector into a host cell, and selecting a cell that produces a multispecific antibody from the host cell into which the vector set has been introduced.

In the present disclosure, the "vector set" means a set of a first expression vector and a second expression vector. The details of the vector set, the first expression vector, and the second expression vector will be described later.

In the present disclosure, in a case of describing common matters between the first expression vector and the second expression vector, the first expression vector and the second expression vector are collectively referred to as an "expression vector".

The host cell may be a prokaryotic cell or a eukaryotic cell. Examples of the prokaryotic cell include a bacterial cell. Examples of the eukaryotic cell include yeast, an insect cell, and a mammalian cell. As the host cell, a eukaryotic cell is preferable, and a mammalian cell is more preferable.

Examples of the bacterial cell include Gram-negative bacterial cells such as Escherichia coli, Salmonella typhimurium, Serratia marcescens, Pseudomonas putida, and Pseudomonas aeruginosa; and Gram-positive bacterial cells such as Bacillus subtilis. As the bacterial cell, Enterobacteriaceae is preferable, and Escherichia coli, particularly a strain B or a strain K12, is more preferable.

Examples of the yeast include budding yeast (Saccharomyces cerevisiae), Pichia pastoris, and Hansenula polymorpha.

Examples of the insect cell include a BmN cell derived from silkworm (Bombyx mori), an Sf9 cell and an Sf21 cell derived from cabbage armyworm (Spodoptera frugiperda), an S2 cell derived from fruit fly (Drosophila melanogaster), and Pv11 cells derived from sleeping chironomid (Polypedilum vanderplanki).

Examples of the mammalian cell include a Chinese hamster ovary cell (CHO cell), a baby hamster kidney cell (BHK cell), human embryonic kidney cell lines (for example, an HEK293 cell), human retinoblastoma-derived cell lines (for example, a PER.C6 cell), and mouse myeloma cell lines (for example, an NS0 cell and an SP2/0 cell).

The host cell is preferably the Chinese hamster ovary cell (CHO cell). Examples of the CHO cell include a CHO-DG44 cell, a CHO-K1 cell, a CHO-DXB11 cell, a CHOpro3⁻ cell, and an established cell derived from these cells.

Examples of a means for introducing the expression vector into a host cell include electroporation, lipofection, microinjection, and cell infection with a viral vector. From the viewpoints of high safety, high introduction efficiency, and low cytotoxicity, electroporation is preferable. In addition, in a case where a plurality of types of expression vectors are introduced into a cell, electroporation is preferable from the viewpoint that the expression vectors are introduced into cells and chromosomes with high uniformity, without bias depending on the type of the expression vector.

The order in which the first expression vector and the second expression vector are introduced into the host cell is not limited. The first expression vector and the second expression vector may be introduced into the host cell together or separately. From the viewpoint of shortening the steps and the period required for producing the target cell, it is preferable that the first expression vector and the second expression vector be introduced into the host cell together.

In a case where the first expression vector and the second expression vector are introduced into a host cell together, a vector solution containing both expression vectors is prepared. In this vector solution, the molar concentration ratio of the first expression vector to the second expression vector is, for example, 1:5 to 5:1, 1:2 to 2:1, or 1:1.

In a case where the first expression vector and the second expression vector are separately introduced into a host cell, a vector solution containing the first expression vector and a vector solution containing the second expression vector are prepared. The molar concentration ratio of the expression vectors of both vector solutions is, for example, 1:5 to 5:1, 1:2 to 2:1, or 1:1.

The expression vector introduced into the host cell may be integrated into the genome of the host cell, may be integrated into an extrachromosomal factor such as a plasmid, or may be contained in the cell as an independent extrachromosomal factor. From the viewpoint of enabling long-term expression of the target multispecific antibody, it is preferable that the expression vector be integrated into the genome of the host cell.

The selection of a cell that produces a multispecific antibody from a host cell into which a vector set has been introduced is performed, for example, by setting a criterion for the expression level and/or the purity of the multispecific antibody and selecting a cell that meets the criterion; and/or selecting a cell having a relatively high expression level and/or the purity of the multispecific antibody. Specifically, for example, the following (a) to (d) are performed.
(a) Adding a selective agent to a culture medium of a host cell.
(b) Performing single-cell isolation on the host cells.
(c) Sampling a part of a culture solution of the single-cell isolated cells, and measuring an expression level and/or a purity of the multispecific antibody.
(d) Selecting a cell in which the expression level and/or the purity of the multispecific antibody is equal to or more than a reference value, and/or selecting a cell in which the expression level and/or the purity of the multispecific antibody is relatively high.

The expression level of the multispecific antibody is the amount of the multispecific antibody itself.

The purity of the multispecific antibody means the proportion (on a mass basis or on a number basis) of the target multispecific antibody in the total amount of the plurality of types of proteins. In the expression of the multispecific antibody, a multimeric protein (for example, a multimeric protein in which a part of a polypeptide is lacking, or a multimeric protein in which a certain polypeptide is replaced with another polypeptide) that is not in the original shape may be generated, and it is desirable that the proportion of the multimeric protein that is not in the original shape be low.

The cell that produces the multispecific antibody may be a transiently expressing cell or a stably expressing cell of the multispecific antibody, and it is preferably a stably expressing cell.

### <Vector Set>

One of the embodiments of the present disclosure is a vector set that is used for producing a cell that produces a multispecific antibody. The vector set is a set of a first expression vector and a second expression vector.

The first expression vector contains all expression cassettes of four types of polypeptides constituting the multispecific antibody. That is, the first expression vector contains an expression cassette of the first H chain, an expression cassette of the second H chain, an expression cassette of the first L chain, and an expression cassette of the second L chain. The expression of the four types of polypeptides is achieved by introducing the first expression vector into the host cell.

The second expression vector contains an expression cassette of a polypeptide of interest (generally, a polypeptide whose expression is desired to be enhanced) among the four types of polypeptides constituting the multispecific antibody. The polypeptide of interest is one type, two types, or three types. That is, the second expression vector contains one type, two types, or three types of expression cassettes selected from the group consisting of the expression cassette of the first H chain, the expression cassette of the second H chain, the expression cassette of the first L chain, and the expression cassette of the second L chain. By introducing the second expression vector into the host cell in addition to the first expression vector, the number of copies of the expression cassette of the polypeptide of interest that is introduced into the host cell increases relatively, and the expression of the polypeptide of interest is enhanced.

A form containing at least an expression cassette of the first L chain and/or an expression cassette of the second L chain is given as an example of the embodiment of the second expression vector, and preferred examples thereof include a form containing at least the expression cassette of the first L chain and the expression cassette of the second L chain. The number of copies of the expression cassette of the first L chain and/or the expression cassette of the second L chain that are introduced into the host cell increases relatively because of the vector set containing the second expression vector, and the expression of the first L chain and/or the second L chain is enhanced. By enhancing the expression of the first L chain and/or the second L chain (preferably the first L chain and the second L chain), the expression level and/or the purity of the multispecific antibody tends to increase.

A form containing at least an expression cassette of a chain having a relatively large molecular weight (the expression cassette of the first H chain and/or the expression cassette of the second H chain) is given as an example of the embodiment of the second expression vector. The number of copies of the expression cassette of the chain having a relatively large molecular weight that is introduced into a host cell increases relatively because of the vector set containing the second expression vector, and the expression of the chain having a relatively large molecular weight (the first H chain and/or the second H chain) is enhanced. The expression level and/or the purity of the multispecific antibody tends to increase by enhancing the expression of the chain having a relatively large molecular weight.

A form containing at least an expression cassette of an H chain having the largest molecular weight (the expression cassette of the first H chain or the expression cassette of the second H chain) is given as an example of the embodiment of the second expression vector. The number of copies of the expression cassette of the H chain having the largest molecular weight that is introduced into a host cell increases relatively because of the vector set containing the second expression vector, and the expression of the H chain having the largest molecular weight (the first H chain or the second H chain) is enhanced. The expression level and/or the purity of the multispecific antibody tends to increase by enhancing the expression of the H chain having the largest molecular weight.

A form containing an expression cassette of an H chain having the largest molecular weight (the expression cassette of the first H chain or the expression cassette of the second H chain), the expression cassette of the first L chain, and the expression cassette of the second L chain is given as an example of the embodiment of the second expression vector. The number of copies of the expression cassette of the H chain having the largest molecular weight, the expression cassette of the first L chain, and the expression cassette of the second L chain that are introduced into a host cell increases relatively because of the vector set containing the second expression vector, and the expression of the H chain having the largest molecular weight (the first H chain or the second H chain), the first L chain, and the second L chain are enhanced. The expression level and/or the purity of the multispecific antibody tends to increase by enhancing the expression of the H chain having the largest molecular weight and the L chain.

A form containing an expression cassette of an H chain (the expression cassette of the first H chain or the expression cassette of the second H chain) which is predicted to have the lowest expression rate from experimental data or a coding sequence, the expression cassette of the first L chain, and the expression cassette of the second L chain is given as an example of the embodiment of the second expression vector. The number of copies of the expression cassette of the H chain having the lowest expression rate, the expression cassette of the first L chain, and the expression cassette of the second L chain that are introduced into the host cell increases relatively because of the vector set containing the second expression vector, and the expression of the H chain having the lowest expression rate (the first H chain or the second H chain), the first L chain, and the second L chain are enhanced. The expression level and/or the purity of the multispecific antibody tends to increase by enhancing the expression of the H chain having the lowest expression level and the L chain.

The sizes of the first expression vector and the second expression vector are not limited.

The size of the first expression vector is, for example, 5,000 base pair (bp) to 50,000 bp, 8,000 bp to 40,000 bp, or 10,000 bp to 30,000 bp.

The size of the second expression vector is, for example, 5,000 base pair (bp) to 50,000 bp, 8,000 bp to 40,000 bp, or 10,000 bp to 30,000 bp.

The expression cassette of a polypeptide contains all nucleic acids required for the expression of the polypeptide. The size and the base sequence of the expression cassette of the polypeptide are not limited. The size of one expression cassette of the polypeptide constituting the multispecific antibody is, for example, 400 base pair (bp) to 4,000 bp, 600 bp to 3,000 bp, or 800 bp to 2,000 bp.

The first expression vector contains an expression cassette of the first H chain, an expression cassette of the second H chain, an expression cassette of the first L chain, and an expression cassette of the second L chain. The four types of expression cassettes contained in the first expression vector may be one or two or more for each type, and may be the same or different between types. That is, in the first expression vector, the number of expression cassettes of the first H chain, the number of expression cassettes of the second H chain, the number of expression cassettes of the first L chain, and the number of expression cassettes of the second L chain are independent of each other, and may be the same or different from each other.

A form in which the number ratio of the four types of expression cassettes is first H chain:second H chain:first L chain:second L chain = 1:1:1:1 is given as an example of the embodiment of the first expression vector.

From the viewpoint of reducing the size of the first expression vector, the first expression vector preferably contains one copy each of the expression cassette of the first H chain, the expression cassette of the second H chain, the expression cassette of the first L chain, and the expression cassette of the second L chain.

The second expression vector contains one type, two types, or three types of expression cassettes selected from the group consisting of the expression cassette of the first H chain, the expression cassette of the second H chain, the expression cassette of the first L chain, and the expression cassette of the second L chain. In a case where the second expression vector contains one type of expression cassette selected from four types, the number of the one type of expression cassette may be one or two or more. In a case where the second expression vector contains two or three types of expression cassettes selected from four types, the number of the two or three types of expression cassettes may be one or two or more for each type, and the two or three types of expression cassettes may be the same or different between types. That is, in a case where two or three types of expression cassettes are selected from the group consisting of the expression cassette of the first H chain, the expression cassette of the second H chain, the expression cassette of the first L chain, and the expression cassette of the second L chain, the number of the two or three types of expression cassettes in the second expression vector is independent of each other, and may be the same or different from each other.

In a case where the second expression vector contains two types of expression cassettes selected from four types, a form in which the number ratio of the expression cassettes is 1:1 is given as an example of the embodiment.

In a case where the second expression vector contains three types of expression cassettes selected from four types, a form in which the number ratio of the expression cassettes is 1:1:1 is given as an example of the embodiment.

From the viewpoint of reducing the size of the second expression vector, the second expression vector preferably contains one copy each of one type, two types, or three types of expression cassettes selected from the group consisting of the expression cassette of the first H chain, the expression cassette of the second H chain, the expression cassette of the first L chain, and the expression cassette of the second L chain.

In the vector set of the present disclosure, from the viewpoint of reducing the size of the expression vector, it is preferable that the first expression vector contain one copy each of an expression cassette of the first H chain, an expression cassette of the second H chain, an expression cassette of the first L chain, and an expression cassette of the second L chain, and that the second expression vector contain one copy each of one type, two types, or three types of expression cassettes selected from the group consisting of the expression cassette of the first H chain, the expression cassette of the second H chain, the expression cassette of the first L chain, and the expression cassette of the second L chain.

The tetrameric antibody having the form shown in Fig. 1 and the pentameric antibody having the form shown in Fig. 2 will be described as an example. In the following description, the first H chain is referred to as "HC1", the second H chain is referred to as "HC2", the first L chain is referred to as "LC1", and the second L chain is referred to as "LC2".

As an example of the embodiment of the vector set that expresses the tetrameric antibody having the form shown in Fig. 1, a form in which the first expression vector contains the expression cassette of HC1, the expression cassette of HC2, the expression cassette of LC1, and the expression cassette of LC2, and the second expression vector contains the expression cassette of LC1 and the expression cassette of LC2 is given. In the present embodiment, the expression of the L chain is enhanced.

More preferred examples of the above-described embodiment include a form in which the first expression vector contains one copy each of the expression cassette of HC1, the expression cassette of HC2, the expression cassette of LC1, and the expression cassette of LC2, and the second expression vector contains one copy each of the expression cassette of LC1 and the expression cassette of LC2.

As an example of the embodiment of the vector set that expresses the pentameric antibody having the form shown in Fig. 2, a form in which the first expression vector contains the expression cassette of HC1, the expression cassette of HC2, the expression cassette of LC1, and the expression cassette of LC2, and the second expression vector contains the expression cassette of LC1 and the expression cassette of LC2 is given. In the present embodiment, the expression of the L chain is enhanced.

More preferred examples of the above-described embodiment include a form in which the first expression vector contains one copy each of the expression cassette of HC1, the expression cassette of HC2, the expression cassette of LC1, and the expression cassette of LC2, and the second expression vector contains one copy each of the expression cassette of LC1 and the expression cassette of LC2.

As an example of the embodiment of the vector set that expresses the pentameric antibody having the form shown in Fig. 2, a form in which the first expression vector contains the expression cassette of HC1, the expression cassette of HC2, the expression cassette of LC1, and the expression cassette of LC2, and the second expression vector contains the expression cassette of LC2 is given. In the present embodiment, the expression of two LC2's in one antibody is enhanced.

More preferred examples of the above-described embodiment include a form in which the first expression vector contains one copy each of the expression cassette of HC1, the expression cassette of HC2, the expression cassette of LC1, and the expression cassette of LC2, and the second expression vector contains one expression cassette of LC2.

As an example of the embodiment of the vector set that expresses the pentameric antibody having the form shown in Fig. 2, a form in which the first expression vector contains the expression cassette of HC1, the expression cassette of HC2, the expression cassette of LC1, and the expression cassette of LC2, and the second expression vector contains the expression cassette of HC1 is given. In the present embodiment, the expression of HC1 having the largest molecular weight is enhanced.

More preferred examples of the above-described embodiment include a form in which the first expression vector contains one copy each of the expression cassette of HC1, the expression cassette of HC2, the expression cassette of LC1, and the expression cassette of LC2, and the second expression vector contains one expression cassette of HC1.

As an example of the embodiment of the vector set that expresses the pentameric antibody having the form shown in Fig. 2, a form in which the first expression vector contains the expression cassette of HC1, the expression cassette of HC2, the expression cassette of LC1, and the expression cassette of LC2, and the second expression vector contains the expression cassette of HC1, the expression cassette of LC1, and the expression cassette of LC2 is given. In the present embodiment, the expression of HC1 having the largest molecular weight and the L chain is enhanced.

More preferred examples of the above-described embodiment include a form in which the first expression vector contains one copy each of the expression cassette of HC1, the expression cassette of HC2, the expression cassette of LC1, and the expression cassette of LC2, and the second expression vector contains one copy each of the expression cassette of HC1, the expression cassette of LC1, and the expression cassette of LC2.

As an example of the embodiment of the vector set that expresses the pentameric antibody having the form shown in Fig. 2, a form in which the first expression vector contains the expression cassette of HC1, the expression cassette of HC2, the expression cassette of LC1, and the expression cassette of LC2, and the second expression vector contains the expression cassette of HC1 and the expression cassette of LC2 is given. In the present embodiment, the expression of HC1 having the largest molecular weight and two LC2's in one antibody is enhanced.

More preferred examples of the above-described embodiment include a form in which the first expression vector contains one copy each of the expression cassette of HC1, the expression cassette of HC2, the expression cassette of LC1, and the expression cassette of LC2, and the second expression vector contains one copy each of the expression cassette of HC1 and the expression cassette of LC2.

The nucleic acid and the base sequence of the base for constructing the expression vector are not limited. Examples of the nucleic acid of the base for constructing the expression vector include a nucleic acid derived from a viral vector, a nucleic acid derived from a non-viral vector, and an artificial nucleic acid. The nucleic acid of the base may be a circular nucleic acid or a linear nucleic acid.

Examples of the nucleic acid derived from a viral vector include a nucleic acid derived from an adenovirus, an adeno-associated virus, a retrovirus, a vaccinia virus, a poxvirus, a lentivirus, a herpes virus, a baculovirus, or a bacteriophage.

Examples of the nucleic acid derived from a non-viral vector include a plasmid.

The expression vector and the expression cassette contain elements required for the expression of a polypeptide, depending on the type of the host cell.

In a case where the host cell is a prokaryotic cell, the expression vector may include, for example, a plasmid stability locus such as a replication origin, a restriction enzyme site, a transcription terminator, and a cer stability sequence.

In a case where the host cell is yeast, the expression vector may include, for example, a promoter, a transcription terminator, a selection marker, and a replication origin.

In a case where the host cell is an insect cell, the expression vector may include, for example, a promoter, a transcription terminator, and a selection marker.

In a case where the host cell is a mammalian cell, the expression vector may include, for example, a promoter, a polyadenylation sequence (for example, a human β-globin polyA sequence, a bovine growth hormone polyA sequence, an SV40 early polyA sequence, or an SV40 late polyA sequence), and a selection marker.

The promoters that can be used in a prokaryotic cell include a promoter disclosed in J. Mol. Biol. 1986; 189(1): 113-30, a phage polymerase promoter, and an Escherichia coli polymerase promoter. Specific examples thereof include T7A1, T7A2, T7A3, λpL, λpR, lac, lacUV5, trp, tac, trc, phoA, and rrnB.

Examples of the promoters that can be used for yeast cells include a gal promoter, an AOX1 promoter, an AOX2 promoter, a GAP promoter, a GAL1 promoter, and a GAL10 promoter.

Examples of the promoters that can be used in insect cells include a polyhedrin promoter, a P10 promoter, an immediate early protein (IE-1) promoter of virus infection, an MT promoter, a COPIA promoter, a CMV promoter, an RSV promoter, an SV40 promoter, a heat shock protein promoter, an OPIE2 promoter, and an actin 5C promoter.

Examples of the promoters that can be used for mammalian cells include virus-derived promoters and housekeeping gene-derived promoters. Examples of the virus-derived promoters include a human CMV promoter, a rat CMV promoter, an SV40 promoter, an RSR-LTR promoter, and an HSK-TK promoter. Examples of the housekeeping gene-derived promoters include an hEF-1α promoter, a Chinese hamster EF-1α promoter, a β-actin promoter, and a mouse phosphoglycerate kinase (mPGK) promoter. A preferred example of the promoter that can be used for mammalian cells is an EF-1α promoter, and a more preferred example thereof is the hEF-1α promoter.

The promoters contained in the expression cassettes of the four types of polypeptides constituting the multispecific antibody may be the same as or different from each other. In an example of the embodiment, all of the expression cassettes of the four types of polypeptides constituting the multispecific antibody contain the same type of promoter. In a preferred example of the embodiment, all of the expression cassettes of the four types of polypeptides constituting the multispecific antibody contain the hEF-1α promoter.

The expression cassette of the polypeptide constituting the multispecific antibody preferably contains a coding sequence of a secretion leader for the purpose of promoting transport or secretion of the expressed antibody polypeptide to the outside of the cell. The secretion leader is one type of signal peptide, and is a signal peptide that induces transport or secretion of a polypeptide to the outside of a cell.

In a case where the expression cassette of the polypeptide constituting the multispecific antibody contains the coding sequence of the secretion leader, the coding sequence of the secretion leader and the coding sequence of the antibody polypeptide are arranged in the same reading frame. Here, the term "arranged in the same reading frame" means that the two coding sequences are arranged such that the secretion leader and the antibody polypeptide can be expressed as one polypeptide. In the expression cassette of the antibody polypeptide, a coding sequence of a linker or spacer may or may not be present between the coding sequence of the secretion leader and the coding sequence of the antibody polypeptide.

A preferred form is a form in which the coding sequence of the antibody polypeptide is arranged downstream of the coding sequence of the secretion leader in the same reading frame. A recombinant polypeptide in which a secretion leader is arranged on the N-terminal side of the antibody polypeptide is expressed from the expression cassette of the present form. A more preferred form is a form in which the coding sequence of the antibody polypeptide is continuously arranged downstream of the coding sequence of the secretion leader in the same reading frame. A recombinant polypeptide in which a secretion leader is bonded to the N-terminal of the antibody polypeptide is expressed from the expression cassette of the present form. Here, the term "downstream" means the arrangement order of two coding sequences, and in a case where both coding sequences are arranged such that the coding sequence B is transcribed after the transcription of the coding sequence A, the coding sequence B is said to be arranged downstream of the coding sequence A.

The secretion leader of the recombinant polypeptide is generally cleaved from the recombinant polypeptide in the process of transport or secretion of the recombinant polypeptide.

Examples of the secretion leader include a fibronectin secretion leader, a collagen secretion leader, and an albumin secretion leader. From the viewpoint of a high secretion rate of the recombinant polypeptide to the outside of the cell, fibronectin secretion leader is preferable.

Examples of the fibronectin secretion leader include a fibronectin secretion leader of an amphibian and a fibronectin secretion leader of a mammal. A fibronectin secretion leader of Xenopus laevis is given as an example of a fibronectin secretion leader of an amphibian. Examples of the fibronectin secretion leader of a mammal include fibronectin secretion leaders of human, rat, mouse, cow, pig, dog, cat, and Chinese hamster, and functional equivalents thereof. The functional equivalent of the fibronectin secretion leader has an identity of 70% or more, preferably 75% or more, more preferably 80% or more, still more preferably 90% or more, and most preferably 95% or more in the amino acid sequence, and has a function of secreting the recombinant polypeptide to the outside of the cell. The sequence identity of the amino acid sequence is calculated using, for example, Basic Local Alignment Search Tool (BLAST) (https://blast.ncbi.nlm.nih.gov/Blast.cgi).

It is preferable to select an originating organism of the fibronectin secretion leader according to the type of the host cell. In a case where the host cell is a human cell, it is preferable to use a human fibronectin secretion leader in the expression cassette. In a case where the host cell is a rat cell, it is preferable to use a rat fibronectin secretion leader in the expression cassette. In a case where the host cell is a CHO cell, it is preferable to use a Chinese hamster fibronectin secretion leader in the expression cassette.

Examples of the embodiment of the fibronectin secretion leader include a human fibronectin secretion leader having an amino acid sequence of SEQ ID NO: 1 and a Chinese hamster fibronectin secretion leader having an amino acid sequence of SEQ ID NO: 2. An example of the coding sequence of SEQ ID NO: 1 is SEQ ID NO: 3. An example of the coding sequence of SEQ ID NO: 2 is SEQ ID NO: 4.

SEQ ID NO: 1: Met Leu Arg Gly Pro Gly Pro Gly Leu Leu Leu Leu Ala Val Gln Cys
Leu Gly Thr Ala Val Pro Ser Thr Gly Ala (MLRGPGPGLLLLAVQCLGTAVPSTGA)

SEQ ID NO: 2: Met Leu Arg Gly Pro Gly Pro Gly Leu Leu Leu Ala Val Leu Cys Leu
Gly Thr Ala Val Arg Cys Thr Glu Ala (MLRGPGPGLLLAVLCLGTAVRCTEA)

SEQ ID NO: 3:
ATGCTGAGAGGCCCTGGACCTGGACTGCTGCTGCTGGCTGTGCAGTGTCTGGGAA
CCGCCGTGCCTTCTACCGGCGCC

SEQ ID NO: 4:
ATGCTCAGGGGTCCGGGACCCGGGCTGCTGCTGGCCGTCCTGTGCCTGGGGACAG
CGGTGCGCTGTACCGAAGCC

A form in which all of the expression cassettes of the four types of polypeptides constituting the multispecific antibody contain the coding sequence of the fibronectin secretion leader is given as an example of the embodiment.

In a case where the host cell is a human cell, it is preferable that all of the expression cassettes of the four types of polypeptides constituting the multispecific antibody contain the coding sequence of the human fibronectin secretion leader.

In a case where the host cell is a CHO cell, it is preferable that all of the expression cassettes of the four types of polypeptides constituting the multispecific antibody contain the coding sequence of the Chinese hamster fibronectin secretion leader.

In a preferred example of the embodiment, all of the expression cassettes of the four types of polypeptides constituting the multispecific antibody contain an hEF-1α promoter, a coding sequence of a fibronectin secretion leader, a coding sequence of an antibody polypeptide, and a polyA sequence, which are operably linked to each other.

The expression vector preferably contains an expression cassette of a selection marker for the purpose of confirming the introduction of the expression vector into a host cell or for the purpose of producing a stably expressing cell.

In the present disclosure, the selection marker is a marker that functions to select a cell into which an expression vector has been introduced, and means a gene integrated into the expression vector and a protein encoded by the gene.

The expression cassette of the selection marker contains all nucleic acids required for the expression of the selection marker. The size and the base sequence of the expression cassette of the selection marker are not limited.

Examples of the selection marker include a protein that exhibits resistance to a selective agent and a gene thereof. Examples of the selective agent include an enzyme inhibitor and an antibiotic.

A DHFR-MTX system is given as an example in which the selective agent is an enzyme inhibitor. In the DHFR-MTX system, the selective agent is methotrexate (MTX), and the selection markers are dihydrofolate reductase (DHFR) and a gene thereof. The DHFR-MTX system is an effective system in host cells (for example, CHO-DG44 cells) that are deficient in the DHFR gene.

A GS-MSX system is given as an example in which the selective agent is an enzyme inhibitor. In the GS-MSX system, the selective agent is methionine sulfoximine (MSX), and the selection marker is glutamine synthetase (GS) and a gene thereof. The GS-MSX system is an effective system in a host cell (for example, a GS knockout CHO cell) that is deficient in the GS gene.

In a case where the selective agent is an antibiotic, an antibiotic-degrading enzyme and an antibiotic resistance gene which is a gene of the antibiotic-degrading enzyme are a selection marker. Examples thereof include a hygromycin resistance gene, a neomycin resistance gene, a puromycin resistance gene, a chloramphenicol resistance gene, a tetracycline resistance gene, an erythromycin resistance gene, a spectinomycin resistance gene, a kanamycin resistance gene, a G418 resistance gene, a bleomycin resistance gene, a zeocin resistance gene, a phleomycin resistance gene, and an ampicillin resistance gene.

In an example of the embodiment of the vector set, the first expression vector contains an expression cassette of a first selection marker, the second expression vector contains an expression cassette of a second selection marker, and the first selection marker and the second selection marker are of different types from each other. The first selection marker is, for example, at least one selected from the group consisting of a dihydrofolate reductase gene, a glutamine synthetase gene, and an antibiotic resistance gene. The second selection marker is, for example, at least one selected from the group consisting of a dihydrofolate reductase gene, a glutamine synthetase gene, and an antibiotic resistance gene.

In an example of the embodiment of the vector set, one of the first expression vector or the second expression vector contains an expression cassette of a dihydrofolate reductase gene or a glutamine synthetase gene, and the other contains an expression cassette of an antibiotic resistance gene. In a preferred example of the expression cassette of the dihydrofolate reductase gene, an mPGK promoter is contained. In a preferred example of the expression cassette of the glutamine synthetase gene, an mPGK promoter is contained. In a preferred example of the expression cassette of the antibiotic resistance gene, a virus-derived promoter is contained.

From the viewpoint of increasing the expression level of the antibody polypeptide from the first expression vector relative to the expression level of the antibody polypeptide from the second expression vector, it is preferable that, in the vector set, the first selection marker is a dihydrofolate reductase gene or a glutamine synthetase gene, and the second selection marker is an antibiotic resistance gene. The expression vector containing the dihydrofolate reductase gene or the glutamine synthetase gene tends to have an increased copy number in a host cell more easily than the expression vector containing the antibiotic resistance gene.

The embodiment of the present disclosure does not exclude the use of a third expression vector other than the vector set (that is, the first expression vector and the second expression vector). The third expression vector is used, for example, to express a polypeptide required for the growth or metabolism of a host cell.

### <Cell transfected with vector set>

One of the embodiments of the present disclosure is a mammalian cell transfected with a vector set. The mammalian cell transfected with the vector set may be a transiently expressing cell or a stably expressing cell of the multispecific antibody, and it is preferably the stably expressing cell.

One of the embodiments of the present disclosure is a CHO cell that has been transfected with a vector set. Specific examples thereof include a CHO-DG44 cell, a CHO-K1 cell, a CHO-DXB11 cell, a CHOpro3⁻ cell, and established cells derived from these cells, which are transfected with the vector set. The CHO cell transfected with the vector set may be a transiently expressing cell or a stably expressing cell of the multispecific antibody, and it is preferably the stably expressing cell.

The mammalian cells and CHO cells transfected with the vector set of the present disclosure are preferably cells in which the total copy number of the expression cassettes of the L chain (the first L chain + the second L chain) is larger than the total copy number of the expression cassettes of the H chain (the first H chain + the second H chain). As a result of this, the expression level and/or the purity of the antibody tends to increase.

The mRNA amount measured by quantitative polymerase chain reaction (quantitative PCR) is useful for estimating the number of copies of the expression cassette present in the cell.

In mammalian cells and CHO cells transfected with a vector set that expresses a tetrameric antibody having the form shown in Fig. 1, in a case where the mRNA amount of the first H chain is set to 1, it is preferable that the mRNA amount of the second H chain be 0.5 to 2, the mRNA amount of the first L chain be 2 to 5, and the mRNA amount of the second L chain be 2 to 5, which are measured by quantitative PCR.

In mammalian cells and CHO cells transfected with a vector set that expresses a pentameric antibody having the form shown in Fig. 2, in a case where the mRNA amount of the second H chain is set to 1, it is preferable that the mRNA amount of the first H chain be 1 to 2, the mRNA amount of the first L chain be 2 to 5, and the mRNA amount of the second L chain be 2 to 10, which are measured by quantitative PCR.

The mammalian cells and CHO cells transfected with the vector set of the present disclosure are cells that express a multispecific antibody, and can be used for producing a multispecific antibody. The mammalian cells and CHO cells transfected with the vector set of the present disclosure can also be used for administration, infusion, or transplantation to mammals.

### <Method for producing multispecific antibody>

One of the embodiments of the present disclosure is a method for producing a multispecific antibody. The method for producing a multispecific antibody includes culturing a cell that produces a multispecific antibody. By culturing the cells, the multispecific antibody is produced in the cells, and the multispecific antibody is accumulated in the culture solution and/or the cells.

The cell used in the method for producing a multispecific antibody is a cell produced by the method for producing a cell according to one of the embodiments of the present disclosure. Since this cell is a cell selected according to the evaluation criteria and/or the relative production performance according to the multispecific antibody, the productivity of the multispecific antibody is excellent.

The culture method for cells and the culture medium composition for producing a multispecific antibody may be selected depending on the type of the host cell. Culture conditions (for example, culture scale, cell density, temperature, and CO₂ concentration) may also be selected depending on the type of host cell.

As an example of the embodiment, the method for producing a multispecific antibody includes recovering the multispecific antibody from a culture solution. Examples of the method of recovering the multispecific antibody from the culture solution include centrifugation, filtration, diafiltration, ion exchange chromatography, affinity chromatography, hydrophobic interaction chromatography, gel filtration chromatography, and high performance liquid chromatography (HPLC). The recovered multispecific antibody is used, for example, in the manufacture of a pharmaceutical composition.

As an example of the embodiments, the method for producing a multispecific antibody includes recovering cells in which the multispecific antibody has accumulated from a culture medium. Examples of a method of recovering cells from a culture medium include centrifugation and filtration. The multispecific antibody accumulates inside or on the surface of a cell according to the properties thereof. The recovered cells are, for example, administered, infused, or transplanted into a mammal.

### <Method for producing cell pool>

One of the embodiments of the present disclosure is a method for producing a cell pool. The method for producing a cell pool includes introducing a vector set into a host cell. The produced cell pool is subjected to selection of cells that produce a multispecific antibody. In the present disclosure, the cell pool means a population of cells having diversity in the number of copies of the expression cassettes of the four types of polypeptides constituting the multispecific antibody.

The scale of the cell pool is not limited, but the cell pool includes, for example, 10 to 10,000 types of clones.

As the host cell used in the method for producing a cell pool, a eukaryotic cell is preferable, a mammalian cell is more preferable, and a CHO cell is still more preferable. Examples of the CHO cell include a CHO-DG44 cell, a CHO-K1 cell, a CHO-DXB11 cell, a CHOpro3⁻ cell, and an established cell derived from these cells.

The cells contained in the cell pool may be transiently expressed cells or stably expressed cells of the multispecific antibody, and are preferably the stably expressed cells.

The cell pool may be maintained by culturing and may be stored by freezing the cells.

### Examples

Hereinafter, the method for producing a cell that produces a multispecific antibody and the vector set will be described in more detail with reference to specific examples. The materials, treatment procedures, and the like shown in the specific examples below can be changed as appropriate without departing from the gist of the present disclosure. The scope of the vector set and the like of the present disclosure should not be construed as being limited by the following specific examples.

### <Construction of expression vector>

An expression vector 1 and an expression vector 2 for expressing a bispecific antibody having a pentamer that has two types of H chains having amino acid sequences different from each other and two types of L chains having amino acid sequences different from each other were constructed.

Hereinafter, the above-described bispecific antibody is referred to as "BiAb2", two types of H chains are individually referred to as "HC1" and "HC2", and two types of L chains are individually referred to as "LC1" and "LC2". HC1, HC2, LC1, and LC2 are collectively referred to as an "antibody subunit".

Fig. 2 is a schematic diagram of BiAb2. BiAb2 has a form in which LC1 and LC2 are bound to HC1 and LC2 is bound to HC2. In the BiAb2, HC1 and LC1 constitute a first epitope binding site, HC1 and LC2 constitute a second epitope binding site, and HC2 and LC2 constitute a third epitope binding site. The epitope recognized by the second epitope binding site and the epitope recognized by the third epitope binding site are common. BiAb2 is a bispecific antibody that binds to the first antigen by the first epitope binding site and binds to the second antigen by the second epitope binding site and/or the third epitope binding site.

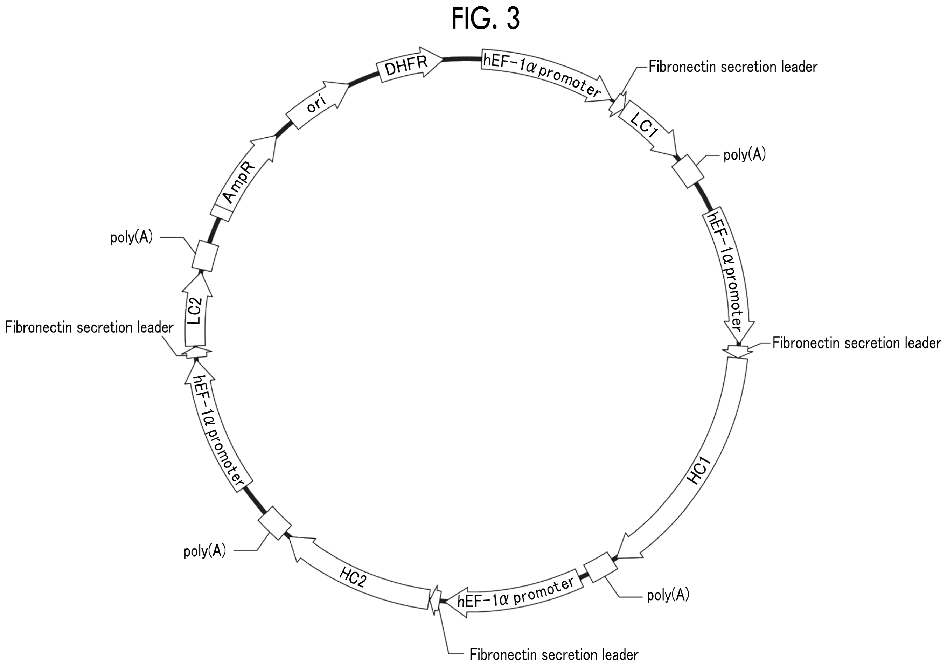

Fig. 3 is a vector map of the expression vector 1. The expression vector 1 is a vector containing one expression cassette of HC1, one expression cassette of HC2, one expression cassette of LC1, one expression cassette of LC2, and one expression cassette of the DHFR gene. Each expression cassette of the antibody subunit contains an hEF-1α promoter, a coding sequence of a fibronectin secretion leader, a coding sequence of an antibody subunit, and a polyA sequence, and the coding sequence of the antibody subunit is continuously arranged downstream of the coding sequence of the fibronectin secretion leader in the same reading frame.

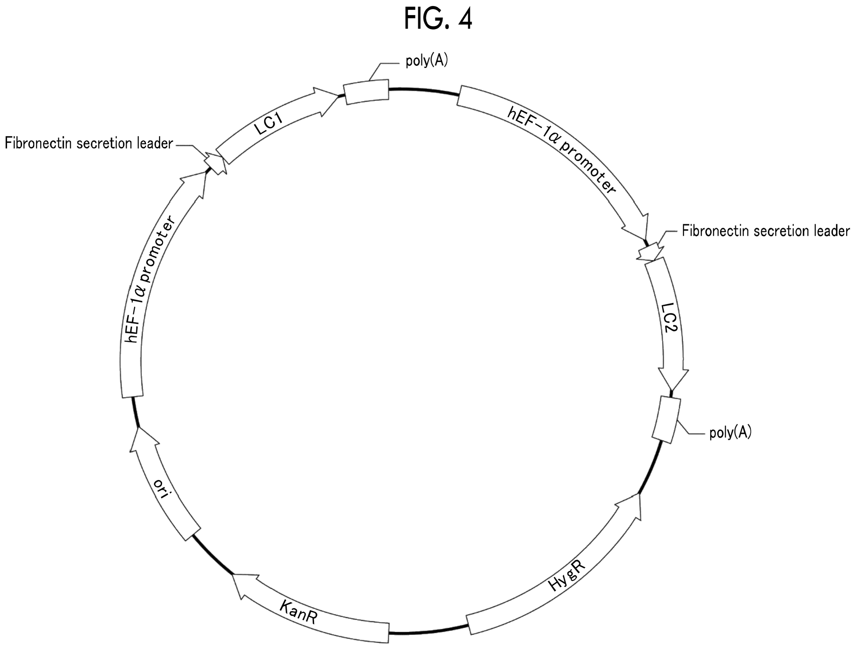

Fig. 4 is a vector map of the expression vector 2. The expression vector 2 is a vector containing one expression cassette of LC1, one expression cassette of LC2, and one expression cassette of a hygromycin resistance gene. Each expression cassette of the antibody subunit contains an hEF-1α promoter, a coding sequence of a fibronectin secretion leader, a coding sequence of an antibody subunit, and a polyA sequence, and the coding sequence of the antibody subunit is continuously arranged downstream of the coding sequence of the fibronectin secretion leader in the same reading frame.

### <Introduction of expression vector into CHO cell>

Either only the expression vector 1 or both the expression vector 1 and the expression vector 2 were introduced into CHO-DG44 cells by an electroporation method.

CHO-DG44 cells into which the expression vector had been introduced were seeded in 10 mL of an OptiCHO culture medium (Lifetechnologies, 12681011) and statically cultured at a temperature of 37°C and in a CO₂ atmosphere of 5% (v/v).

One day after the introduction of the expression vector, methotrexate was added as a selective agent to the CHO-DG44 cells into which only the expression vector 1 had been introduced, and methotrexate and hygromycin B were simultaneously added as selective agents to the CHO-DG44 cells into which the expression vector 1 and the expression vector 2 had been introduced.

14 days after the introduction of the expression vector, the culture volume was expanded to 20 mL, the cell culture was transferred to a 125 mL flask for shaking culture, and subjected to shaking culture.

### <Selection of BiAb2-producing cells>

The cells shaken and cultured were dispensed into a 96-well plate by one cell per well using a droplet cell sorter, and statically cultured at a temperature of 37°C and in a CO₂ atmosphere of 10% (v/v). After culturing for 14 days, the culture supernatant was collected, and the antibody concentration was measured using an intermolecular interaction analyzer Octet Qke (Sartorius). Clones with higher antibody concentrations were selected, cultured in a 24-well plate, and then cultured in a bioreactor tube for scale-up. In this way, cells producing BiAb2 were produced.

The BiAb2 production cells produced by the introduction of only the expression vector 1 are referred to as BiAb2-QGV cells. The BiAb2 production cells produced by the introduction of the expression vector 1 and the expression vector 2 are referred to as BiAb2-QGV/DGV cells.

### <Production of BiAb2>

The BiAb2-QGV cells and the BiAb2-QGV/DGV cells were each suspended in 40 mL of a basal culture medium, transferred to a 125 mL flask for shaking culture, and subjected to shaking culture with feeding at a temperature of 37°C and in a CO₂ atmosphere of 5% (v/v) at a rate of 140 rpm. From the 3rd day to the 13th day after the start of the culture, a certain amount of the feed culture medium was added every day. Sampling was performed every 1 to 3 days, and the cell density, the culture solution components, and the antibody concentration were measured. On the 14th day after the start of the culture, the culture solution was collected, the cells and cell debris were removed using a depth filter (pore size of 0.22 µm) to obtain a culture supernatant.

The antibody concentration in the culture supernatant was measured by liquid chromatography using a Protein A column. The antibody was purified from the culture supernatant using a Protein A column, and the quantification of the BiAb2 and the quantification of the mispaired antibody (antibody in which the H chain was only the HC1 and antibody in which the H chain was only the HC2) were performed using a CE-SDS analyzer PA800 Plus (SCIEX) to determine the purity of BiAb2. Here, the purity is BiAb2/(BiAb2 + mispaired antibody) × 100 (%). Table 1 shows measurement results for the clone having the highest antibody concentration. Qp shown in Table 1 is the amount of antibody produced per day and per cell, and is the amount of antibody produced, excluding the mispaired antibody.

The mRNA amount of the antibody subunit in each clone was measured by quantitative PCR. Table 2 shows measurement results for the clone having the highest antibody concentration. The numerical values shown in Table 2 are relative values in a case where the mRNA amount of each antibody subunit in the BiAb2-QGV cell is set to 1.

**[Table 1]**

| Clone | Qp [pg/cell/day] | Desired BiAb [%] |
|---|---|---|
| BiAb2-QGV | 11.9 | 67.3 |
| BiAb2-QGV/DGV | 16.6 | 76.5 |

**[Table 2]**

| Clone | Relative mRNA expression level (BiAb2-QGV=1) | | | |
|---|---|---|---|---|
| | HC1 | HC2 | LC1 | LC2 |
| BiAb2-QGV | 1 | 1 | 1 | 1 |
| BiAb2-QGV/DGV | 1.2 | 1.0 | 2.0 | 3.0 |

Since the BiAb2-QGV cells are cells transfected with the expression vector 1, it is considered that the number ratio of the expression cassettes contained in the cells is HC1:HC2:LC1:LC2 = 1:1:1:1.

Since the BiAb2-QGV/DGV cells are cells transfected with the expression vector 1 and the expression vector 2, it is considered that the number ratio of the expression cassettes contained in the cells is HC1:HC2:LC1:LC2 = 1:1:more than 1:more than 1.

This was supported by the relative expression levels of mRNAs shown in Table 2. That is, in the BiAb2-QGV/DGV cells, HC1 was 1.2 times, HC2 was 1.0 times, LC1 was 2.0 times, and LC2 was 3.0 times as much as those in the BiAb2-QGV cells.

It is presumed that the difference in the relative expression level of mRNA of LC1 and LC2 in the BiAb2-QGV/DGV cells is due to a difference in transcription efficiency of each expression cassette.

As shown in Table 1, the Qp (antibody productivity per cell) of the BiAb2-QGV cells was 11.9 pg/cell/day, the Qp of the BiAb2-QGV/DGV cells was 16.6 pg/cell/day, and the Qp of the BiAb2-QGV/DGV cells was 1.4 times that of the BiAb2-QGV cells. It was presumed that the excessive expression levels of LC1 and LC2 contributed to the Qp (1.4 times that of the BiAb2-QGV cells) in the BiAb2-QGV/DGV cells.

CHO cells having a high expression level and a high purity of BiAb2 were selected from CHO cells transfected with the vector set of the expression vector 1 and the expression vector 2, whereby it was possible to produce CHO cells having excellent productivity of BiAb2.

All documents, patent applications, and technical standards described in the present specification are incorporated herein by reference to the same extent as in a case of being specifically and individually noted that individual documents, patent applications, and technical standards are incorporated herein by reference.

The disclosure of JP2023-023760 filed on February 17, 2023 is incorporated in the present specification by reference in its entirety.
[Sequence list] International application 23F00050WlJP24005086_2.xml based on International Patent Cooperation Treaty

## Claims

1. A method for producing a cell that produces a multispecific antibody having four types of polypeptides, that is, a first H chain, a second H chain, a first L chain, and a second L chain, the method comprising:
introducing the following vector set into a host cell; and
selecting a cell that produces a multispecific antibody from the host cell into which the vector set has been introduced,
the vector set being a set of a first expression vector and a second expression vector,
in which the first expression vector comprises an expression cassette of the first H chain, an expression cassette of the second H chain, an expression cassette of the first L chain, and an expression cassette of the second L chain, and
the second expression vector comprises one type, two types, or three types of expression cassettes selected from the group consisting of the expression cassette of the first H chain, the expression cassette of the second H chain, the expression cassette of the first L chain, and the expression cassette of the second L chain.

2. The method for producing a cell according to claim 1,
wherein the second expression vector comprises the expression cassette of the first L chain and the expression cassette of the second L chain.

3. The method for producing a cell according to claim 1,
wherein the multispecific antibody is a pentamer in which the first L chain and the second L chain are bound to the first H chain and the second L chain is bound to the second H chain.

4. The method for producing a cell according to claim 1,
wherein the first expression vector comprises one copy each of the expression cassette of the first H chain, the expression cassette of the second H chain, the expression cassette of the first L chain, and the expression cassette of the second L chain, and
the second expression vector comprises one copy each of one type, two types, or three types of expression cassettes selected from the group consisting of the expression cassette of the first H chain, the expression cassette of the second H chain, the expression cassette of the first L chain, and the expression cassette of the second L chain.

5. The method for producing a cell according to claim 1,
wherein all of expression cassettes of polypeptides constituting the multispecific antibody comprise the same type of promoter.

6. The method for producing a cell according to claim 1,
wherein the first expression vector further comprises an expression cassette of a first selection marker, and
the second expression vector further comprises an expression cassette of a second selection marker.

7. The method for producing a cell according to claim 1,
wherein the host cell is a mammalian cell.

8. The method for producing a cell according to claim 1,
wherein the host cell is a CHO cell.

9. A method for producing a multispecific antibody, comprising:
culturing a cell produced by the method for producing a cell according to any one of claims 1 to 8.

10. A vector set that expresses a multispecific antibody having four types of polypeptides, that is, a first H chain, a second H chain, a first L chain, and a second L chain,
wherein the vector set is a set of a first expression vector and a second expression vector,
the first expression vector comprises an expression cassette of the first H chain, an expression cassette of the second H chain, an expression cassette of the first L chain, and an expression cassette of the second L chain, and
the second expression vector comprises one type, two types, or three types of expression cassettes selected from the group consisting of the expression cassette of the first H chain, the expression cassette of the second H chain, the expression cassette of the first L chain, and the expression cassette of the second L chain.

11. The vector set according to claim 10,
wherein the second expression vector comprises the expression cassette of the first L chain and the expression cassette of the second L chain.

12. The vector set according to claim 10,
wherein the multispecific antibody is a pentamer in which the first L chain and the second L chain are bound to the first H chain and the second L chain is bound to the second H chain.

13. The vector set according to claim 10,
wherein the first expression vector comprises one copy each of the expression cassette of the first H chain, the expression cassette of the second H chain, the expression cassette of the first L chain, and the expression cassette of the second L chain, and
the second expression vector comprises one type, two types, or three types of expression cassettes, each in a single copy, selected from the group consisting of the expression cassette of the first H chain, the expression cassette of the second H chain, the expression cassette of the first L chain, and the expression cassette of the second L chain.

14. The vector set according to claim 10,
wherein all of expression cassettes of polypeptides constituting the multispecific antibody comprise the same type of promoter.

15. The vector set according to claim 10,
wherein the first expression vector further comprises an expression cassette of a first selection marker, and
the second expression vector further comprises an expression cassette of a second selection marker.

16. A mammalian cell transfected with the vector set according to any one of claims 10 to 15.

17. A CHO cell transfected with the vector set according to any one of claims 10 to 15.

18. A method for producing a cell pool, the method comprising introducing the vector set according to any one of claims 10 to 15 into a host cell.

19. The method for producing a cell pool according to claim 18,
wherein the host cell is a mammalian cell.

20. The method for producing a cell pool according to claim 18,
wherein the host cell is a CHO cell.
